# EUROPEAN PATENT APPLICATION

(11) **EP 1 897 486 A1**
(43) Date of publication of application: **12.03.2008**
(21) Application number: 06120413.7
(22) Date of filing: 11.09.2006
(51) Int. Cl.: A61B 5/00, G01N 21/35

(54) **Optical blood analyte monitor**

(71) Applicant: FOSS Analytical AB, 263 21 Höganäs (SE)
(72) Inventor: Wihlborg, Nils, 25238, Helsingborg (SE)
(74) Representative: Hilton, Charles Paul

(57) **Abstract**

An optical blood analyte monitor (2) comprises a near infra-red light source and a complementary detection (6) means; a component analyser (12) having access to a chemometric model linking optical spectral features to a level of blood analyte of interest and configured to apply the model to signals received from the detection (6) means and a tilting filter arrangement (8) having a plurality of optical interference filters (20a..e;56a..d), each filter being tiltable to vary a wavelength of incident light from the source transmitted there through. The light source comprises a plurality of light emitters (4a..e) each being arranged to emit light along a different associated light-path (16a..e) in which is located an associated different one of the plurality of the interference filters (20a..e) and towards a same analysing region (18) at which a tissue volume (finger for example) containing a blood sample to be analysed is located in use.

## Description

The present invention relates to an optical blood analyte monitor and in particular to a non-invasive blood glucose monitor.

It is known to use optical analysers to monitor blood analytes (for example blood gases, alcohol or glucose) and in particular blood glucose levels in diabetics. Diabetes is a metabolic disorder caused by a body's failure to either produce insulin or use insulin effectively. The hormone, insulin, enables a body to utilize glucose. Studies have indicated that frequent monitoring and control of blood glucose levels reduces the possibility of serious complications in diabetics by fifty to sixty percent.

Probably the most common method for a diabetic person to monitor blood glucose levels is invasive and requires the direct analysis of a blood sample taken from the person. The blood sample is typically obtained by pricking the person's finger. The sample is then analyzed using a monitor that employs a chemically treated strips which either indicates by color the glucose level in the sample being tested or produces a reaction that may be used in an electrical measurement of the glucose level. Because these techniques require at least a finger prick to obtain a blood sample they tend to discourage regular use due to the inconvenient and painful nature of drawing blood through the skin prior to analysis. They are therefore not best suited to the frequent monitoring that has been shown to be beneficial in reducing the long term ill effects of diabetes.

Other, non-invasive, methods and apparatus for direct determination of blood glucose utilizing optical spectroscopic measurements in the visible (380 to 780 nm) and/or near-infrared (780 to 2500 nm) regions of the electromagnetic spectrum are known in which the incident light is partially absorbed and scattered by constituents of the tissue prior to being reflected back to a detector. The detected light thus contains quantitative information that is based on the known interaction of the incident light with components of the body tissue including water, fats, protein, and glucose.

These optical methods and apparatus rely on the detection of the magnitude of light attenuation caused by the absorption fingerprint of blood glucose as represented in the targeted tissue volume. It is known, from for example US 5,529,755 of Higashio et al., to employ optical radiation in one or more of the wavelength bands centered around 1575 nm, 1765nm, 21 00nm and 2270 nm or a broad band between 1560 nm and 1760 nm or between 2080nm and 2230nm in order to detect characteristic absorption by blood glucose.

The signal due to the absorption of glucose is extracted from the spectral measurement using one or more mathematical (chemometric) models. The models are developed through the process of calibration on the basis of a calibration set of spectral measurements and associated known reference blood glucose values typically obtained using known direct analysis methods.

One such optical blood glucose monitor and method of analysis is disclosed in US 6,124,134 of Stark. Here electromagnetic energy from a tungsten halogen source and covering a multiplicity of wavelength bands within a wavelength range from 380 nm to 2500 nm is directed into a person's tissue containing blood. Portions of the energy representative of both the source energy and energy after interacting with material within the tissue are collected by means of a diode array spectrophotometer. The portions are converted into electrical signals representative of the intensities of the respective portions in each of the multiplicity of wavelength bands which themselves are a function of the fractional portion of the energy in each of the wavelength bands absorbed and scattered by the material in the measurement volume of the tissue. Selected groups of the data signals are processed by a signal processor in accordance with chemometric models developed from analysis of such data signals together with known values of the analytes derived from measurements on a calibration set of samples larger in number than the number of wavelength bands included in the set of the selected groups of data signals to develop analyte signals representative of the amounts of glycated compounds for which chemometric models have been developed and utilized.

Another non-invasive optical blood glucose monitor is disclosed in US 20030086074 of Braig et al in which appropriate portions of the selected wavelength regions are selected using either a filter wheel, containing an array of bandpass filters of differing wavelength transmission properties and which are rotated sequentially into the optical path of radiation to be detected by a detector where they are held stationary during a measurement or using an interferometer or using an electrically tunable filter.

In the aforementioned prior art optical blood monitors the optical systems which are employed to generate spectral information are relatively expensive and/or mechanically complex.

So-called 'tilting filter' arrangements may be employed in optical monitors to generate the required narrow bandwidths using a broad band source in a relatively inexpensive and mechanically simple manner.

An optical analyser incorporating a tilting filter arrangement is disclosed in US 4,037,970, the contents of which is incorporated herein by reference. In this analyser a plurality (here three) of narrow band pass interference filters are mounted in a paddle-wheel configuration such that the filters are rotated in sequence into a light-path between a single broad band light source (here a tungsten filament lamp) and an analysing region in which a test sample to be analysed is located in use. Each filter of the plurality is selected to permit the passage of a different, narrow wavelength band and so in order to collect the necessary optical data the paddle wheel is made to describe complete rotations. The rotation of the paddle-wheel arrangement serves also to effect a tilting of the filter as it is swept through the light path. As the angle of incidence of light on the filter varies there is a concomitant variation in the wavelength of the light transmitted through the filter. Thus, as each filter is rotated through the light-path the wavelength of light at the analysing region is swept through a narrow range of values particular to each filter. However, each filter may only provide wavelength variations through a limited degree of tilting and thus during the majority of the rotation of the paddle-wheel little or no relevant optical data can be collected.

A further optical analyser incorporating a tilting filter arrangement is disclosed in US 4,082,464, the contents of which is incorporated herein by reference. In this analyser the paddle-wheel arrangement is replaced by a drum arrangement. A plurality (here six) of interference filters are mounted on a wheel in a drum arrangement. As the wheel rotates then the filters are rotated in sequence through the light-path between a single broad band light source and an analysing region with a concomitant variation in the wavelength of light that is transmitted through the filter. In addition to being able to accommodate an increased number of interference filters the angular position of each filter with respect to the wheel can be easily adjusted to thereby adjust the wavelength region transmitted as the filter rotates through the light-path. However, as with the aforementioned analyser, complete rotations of the wheel remain necessary in order to collect the relevant optical data.

One further problem associated with the known optical analysers is that the broad band light source generates significant heat that must be dissipated in the filters and in the tissue of the person the blood of whom is being analysed. Moreover, the filters of the tilting filter arrangement must be designed so as to block the majority of the wavelengths emitted by the source which increases the cost of such filters and also increases the heat to be dissipated by these filters.

An aim of the present invention is to provide a relatively low cost tilting filter optical blood analyte monitor in which at least a one of the above identified problems is alleviated.

According to a first aspect of the present invention there is provided an optical blood analyte monitor as described in and characterised by the present Claim 1. The use of a plurality of light emitters permits the wavelength spectrum output by each emitter and incident on the associated filter to be reduced. This then reduces the heat dissipation requirements of each filter. Additionally, the emission wavelength profile of each emitter or groups of emitters of the plurality may be made much narrower than the broad band source, usefully tailored to the components of the blood to be analysed, thus reducing the band pass requirements of the interference filters of the monitor and allowing less costly filters to be employed.

Moreover, such a use of a plurality of light emitters can reduce the need to re-calibrate the monitor on replacement of a light emitter since by arranging for a group of two or more of the plurality of light emitters to have substantially the same emission wavelength profile then tissue may be illuminated with an average illumination contributed by all emitters of the group. Thus replacement of a single emitter of the group has less effect on the illumination reaching the tissue.

Simultaneous tilting allows a single drive mechanism to be employed for tilting all filters, thereby reducing constructional complexity and production costs.

Usefully, a light pipe may be provided to collect light from the analysing region and conduct it to a light sensor. Advantageously, the light pipe may be formed of a hollow bodied axle element of the filter arrangement. The axle is preferably produced by injection moulding or other known casting technique and may optionally also have integrated a carrier arrangement for use in tilting the filters. This technique facilitates low cost, high volume production of the tilting filter arrangement optionally having a reduced number of separate components.

Advantageously, each filter of the plurality of filters is reciprocatively tiltable. Movement of the filters may therefore be restricted to substantially that required to achieve a desired variation in the wavelength of light from the source which is present at the analysing region. This permits a faster response and a more rapid data acquisition than if the filters were made to describe complete rotations.

These and other advantages will become apparent from a consideration of the following description of an exemplary embodiment of the invention made with reference to the figures of the accompanying drawings, of which:

Figs. 1 show (a) a first embodiment of an optical blood analyte monitor according to the present invention and (b) cooperation between the detector and the filter arrangement of Fig. 1 (a);

Fig. 2 shows a part sectional view of the tilting filter arrangement of Fig. 1;

Fig. 3 shows in greater detail the drive arrangement of the tilting filter arrangement of Figs. 1 and Fig. 2; and

Fig. 4 shows a second embodiment of an optical blood analyte monitor according to the present invention.

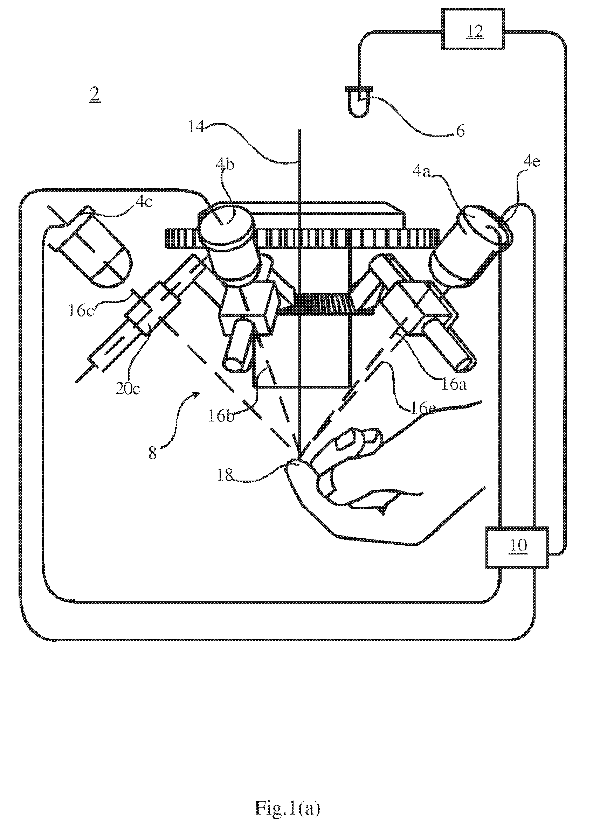

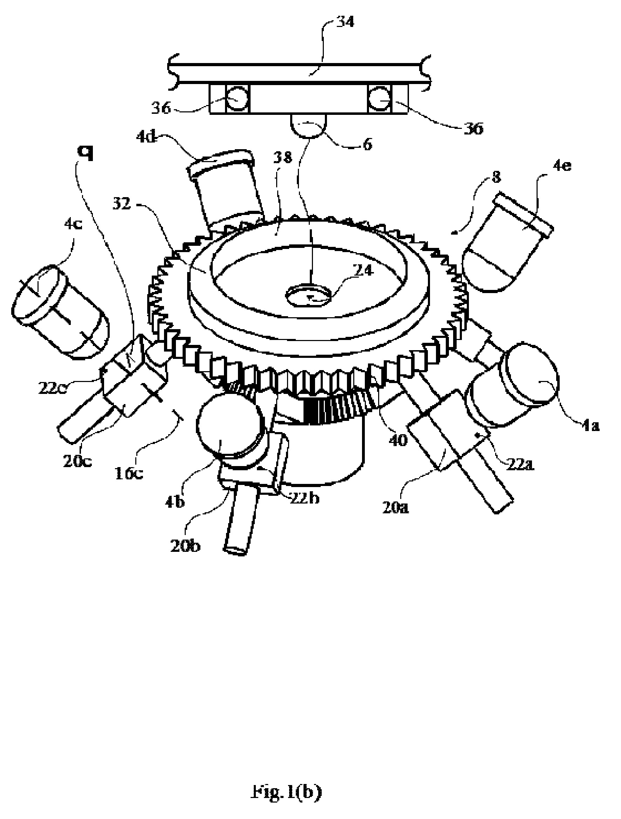

Considering now Fig. 1 (a), a non-invasive blood analyte monitor 2 is shown generally to comprise a near infra-red light source having a plurality (here five are shown) of light emitters 4a...e; a complementary detection means 6 and a tilting filter arrangement 8.

A control unit 10 is provided in the present embodiment for controlling the energisation of each emitter 4a...e and is also operably connected to a computer 12 from which control instructions are sent to the control unit 10 and which is operably connected to receive output, such as indicative of an intensity of light incident at the detection means 6, from the detection means 6.

The computer 12, in the present embodiment, also serves as a data analyser for the analysis of the output to determine a blood glucose level which may then be conveniently provided as audio/visual information for a user, perhaps being displayed on a dedicated display unit (not shown) of generally known type, preferably integral with the monitor. To this end the computer 12 is provided access to a chemometric model linking spectral features to blood glucose level. Indeed any analyte of interest having characteristic optical absorptions may be determined by construction of a suitable chemometric model.

The computer 12 is in the present embodiment configured to hold in memory a calibration model based on one of a number of known regression techniques applied to spectral measurements of a large number of samples in which the blood glucose level (or other analyte of interest) is known and is varied between samples so as to extend across the expected range of blood glucose levels.

Generally known calibration methods capable of simultaneously using measurements from a very large number of wavelengths, sometimes identified as full-spectrum methods, may be employed. Such methods typically employ known multivariate mathematical techniques such as partial least squares (PLS) or multiple linear regression (MLR) or principle component analysis (PCA) techniques. These methods, which are capable of analyzing rather complex materials, have a number of inherent advantages (e.g., signal averaging and improved outlier detection) over methods that use relatively few wavelengths. While still allowing for overlapped spectra of various components, the capability of using many wavelengths eliminates the need for wavelength selection and the implicit requirement of knowledge of the spectra of interfering components. The number of potential spectral wavelengths available to use with a full-spectrum method is often very large, perhaps thousands. Thus, with full-spectrum methods, all available wavelengths within some broad range are typically utilized.

In another embodiment the computer 12 has access to a library of calibration spectra that may be held in a memory associated with the computer 12. The computer 12 is configured to select a g roup of calibration spectra which most closely resembles the spectrum to be analysed and to generate, from those selected calibration spectra, a calibration model which is then applied in the computer 12 to determine the glucose level from the spectrum to be analysed.

In the present embodiment and by way of example only, each emitter 4a...e consists of a light emitting diode (LED) having a narrow (for example, of the order of 100nm) wavelength band emission profile that together cover desired portions of the near infra-red wavelength region between 780 nm to 2500nm. This, for blood glucose monitors preferably includes wavelength bands extending over some or all of the wavelength region between 1000nm and 2000nm. In some embodiments one or more LEDs may be selected having near infra-red emissions in regions not well absorbed by the blood glucose, or any material making up the tissue sample being illuminated. Light which is detected after interaction with the tissue sample may then be used as a reference, employed in the optical calibration of the monitor according to the present invention. The detection means 6 may include a known InGaAs solid state detector.

These emitters 4a...e are arranged angularly spaced apart around a central axis 14 and each is orientated to provide a different associated light path (represented generally by dashed lines 16a,b,c and e) all of which intersect, here approximately at the central axis 14 in what in the present embodiment is an analysing region 18. In use, it is intended that the appropriate body part is located analysing region 18 so as to be capable of being illuminated with light from any emitter 4a...e. Although use of the finger tip as the body part is illustrated in Fig. 1 (a) it will be appreciated that the ear lobe or other site offering convenient access may be substituted.

Considering now also Fig. 1 (b), the tilting filter arrangement 8 comprises a plurality of interference filters 20 a...e, each one selected to have a different narrow band pass (in the present example employing the LED's described above, of the order of 10nm) adapted for its associated emitter 4a...e. Each filter (20c, for example) is located in a light path (16c,for example) of the associated emitter (4c, for example) and is tiltable to vary an angle of incidence ? of light from the associated emitter 4c on a face (22c, for example) of the filter 20c . In this manner and as known in the art the wavelength of the incident light that is transmitted by the filter 20c may be varied as the angle of incidence ? is varied. The same will of course be true for all filters 20a..e and associated emitter 4a..e combinations.

The detection means 6 is here illustrated as comprising a single sensor that in use is positioned (shown by the arrow in Fig. 1 (b)) to monitor light from the LEDs after it is reflected from a body portion (not shown) which is here to be located in the analysing region 18. It will be appreciated that the detection means 6 may be configured to additionally or alternatively monitor light from the LEDs after it is transmitted through the sample, without departing from the invention as claimed.

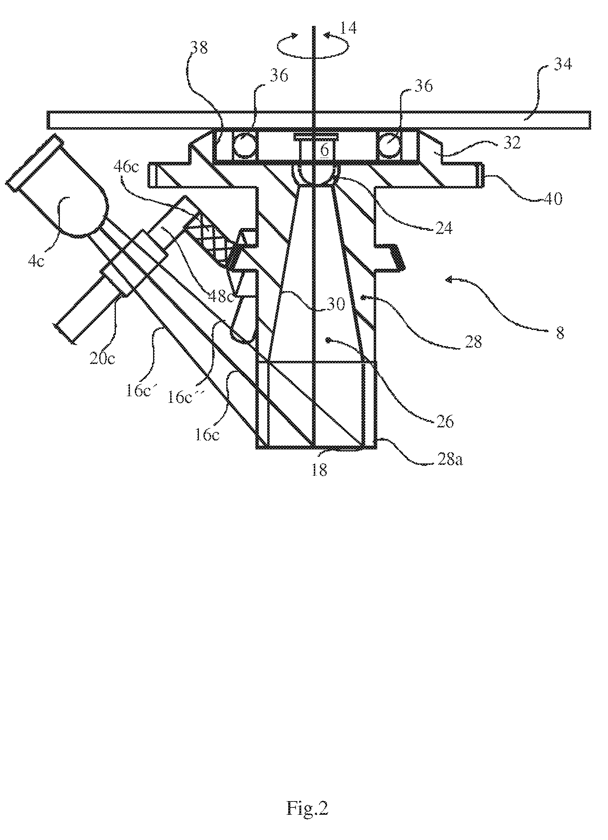

In the present embodiment, as shown in Fig. 1 (b) and Fig. 2, the detector means 6 is intended to be positioned in an opening 24 of a through bore 26 that extends axially along a body portion 28 of the tilting filter arrangement 8. The through bore 26 is optionally provided with a light reflecting internal surface 30 and forms a light pipe for the channelling of light to the detection means 6 after its interaction with the body part in the analysing region 18. In the present embodiment the body portion 28 further comprises a section 28a formed of material which is transparent to the radiation emitted from the emitters 4a..e and which terminates at the analysing region 18. In this way the positioning of the body part in the analysing region 18 is facilitated as it simply may be pressed against the end of the transparent section 28a.

The body portion 28 is here provided with a lip 32 which is intended to form a part of a light tight housing for the detection means 6. A complementary lid 34 is also provided to complete the light tight housing and is here includes bearings, such as a wheel race 36 that engages with an internal surface 38 of the lip 32 so that the lid 34 will remain stationary as the body portion 28 rotates about the axis 14. In the present embodiment the lid 34 also acts as a support for the detector means 6 and may be formed of a printed circuit board holding other electronic components of the analyser 2. Also provided on the body portion 8 is a toothed drive wheel 40 intended for engagement with a complementary toothed wheel of a drive system, such as a stepper motor based system (not shown), which in operation is intended to cause the body portion 28 to rotate, preferably describing an oscillatory motion, about the central axis 14, as illustrated by the double headed arrow in Fig 2.

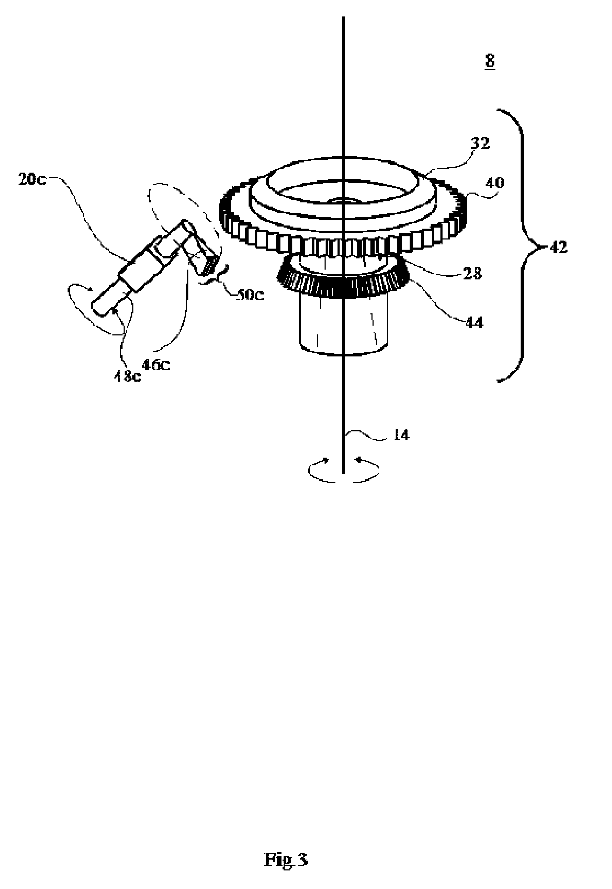

Considering now Fig.3, the tilting filter arrangement 8 of Figs. 1 and Fig. 2 is shown in greater detail and for ease of understanding it is illustrated as having only one filter 20c.

In the present embodiment, the filter arrangement comprises an axle 42 having the cylindrical body portion 28 extending along the rotational axis 14. At one end of the body portion 28, distal the analysing region (not shown), there is provided the lip 32 and the toothed drive wheel 40. A carrier, here in the form of a toothed gear wheel 44 is located about the periphery of the body portion 28 and is presently also included as an integral part of the axle 42. It is envisaged that the axle 42 may be manufactured as a single item, typically using conventional moulding techniques, such as injection moulding. This facilitates the low cost volume production of the filter arrangement 8 employing a minimum of separate parts.

Each filter 20c, say, is provided in mechanical connection with an associated follower, here in the form of a toothed gear wheel 46c, which engages with and is moved, here rotated, by the carrier gear wheel 44 as the axle 42 rotates. In the present embodiment each filter 20c is mounted on a shaft 48c of the associated gear wheel 46c to tilt as the gear wheel 46c (and hence the shaft 48c) rotates and thereby vary the angle of incidence, ?, of light at the filter 20c whilst always remaining in the light path (16c say of Figs. 1 and Fig. 2)as the axle 42 rotates.

It is preferable that the axle 42 and thus the gear wheel 46c is oscillated through only an arc of a circle sufficient to achieve a desired reciprocative tilting movement of the associated filter 20c, preferably but not essentially, about a position where the light is incident substantially perpendicular to a face (22c in Fig. 1(b) of the filter 20c.

In this manner the wavelength of light from an associated emitter that will be incident at the analysing region may be swept through a desired range first in one wavelength direction and then in the opposite wavelength direction.

In this case, and as illustrated in Fig. 3, the follower gear wheel 46c need only comprise a restricted segment 50c of a circle (broken line construction). It will be appreciated that the same is also true for the carrier gear wheel 44. However it is convenient to provide the carrier gear wheel 44 as a continuous gear wheel since it is to engage each of the plurality of follower gear wheels at different locations about the circumference of the body 28.

It will also be appreciated that a detection means 6 should be selected having wavelength response characteristics matching those emission wavelength characteristics of the emitters used and it is envisaged that multiple sensors may be used, particularly in circumstances where there is a large variation in the emission spectral regions of the emitters 4a..e that constitute the light source of the optical analyser 2. The detection means 6 may also be arranged to detect light after its transmission through the sample. Suitably, the detection means 6 may be located to along the axis 14 beyond the body portion 28 such that the analysis region 18 is situated between the body portion 28 and the detection means 6. In this configuration the body portion 28 need not be hollow and will form a solid rotatable axle supporting the carrier 44 and the drive wheel 40.

In one version of this first embodiment of the present invention it is envisaged that the emission wavelength band of each emitter is different and that the wavelength bands together cover portions of the visible and infra-red wavelength regions and are selectably, typically sequentially, energisable dependent on the sample being analysed. In this manner a general purpose analyser may be provided that can analyse a wide variety of samples.

It is also envisaged that a further version of this first embodiment of the present invention may be provided having two or more emitters of the plurality 4a..e that have substantially the same emission wavelength band and which are energised to simultaneously illuminate a sample. In this manner an 'average' illumination of the sample is provided which is relatively insensitive to changes of individual emitters. Thus an optical analyser configured in this manner need not be re-calibrated each time an emitter is replaced.

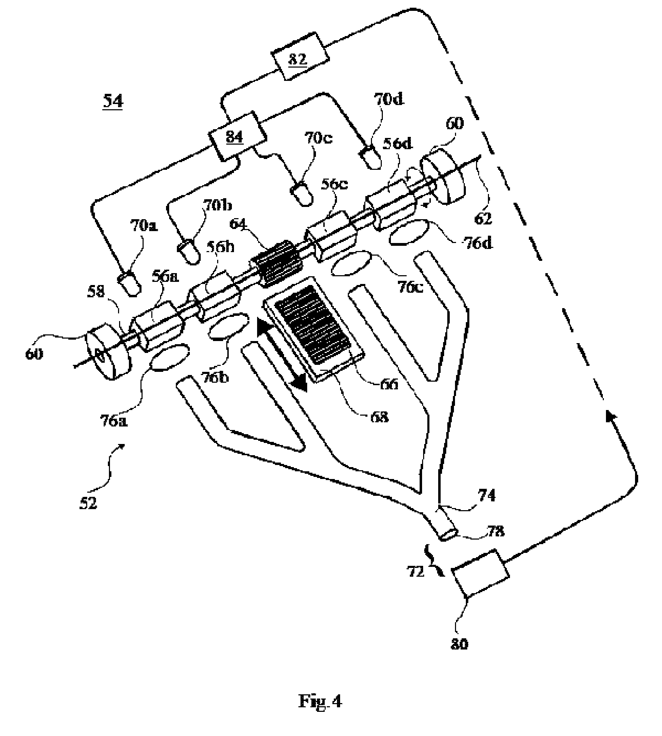

Considering now Fig. 4, a second embodiment of a tilting filter arrangement 52 is illustrated together with relevant components of a second optical analyser 54. Each of a plurality of interference filters 56a..d of the tilting filter arrangement 52 is ganged on a shaft 58 for simultaneous tilting movement as the shaft 58 rotates. The shaft 58 is journalled in bearings 60 for rotation about an axis 62. A toothed follower 64 is formed about at least a portion of the circumference of the shaft 58 and is adapted for engagement with a complementary carrier portion 66 provided on an underside of a drive-plate 68.

In use, the drive-plate 68 is reciprocatively translated (illustrated by the heavier double-headed arrow)to effect a corresponding reciprocal rotation of the shaft 68 (illustrated by the lighter double-headed arrow). In turn, all filters 56a..d are simultaneously caused to execute a reciprocative tilting motion. This tilting motion serves to vary an angle of incidence of light at a surface of an associated filter of the plurality of filters 56a..d whilst each filter 56a..d remains in the light path of the associated emitter 70a..d at all times. The plurality of light emitters 70a..d constitute a light source of the optical analyser 54. In the present embodiment each light emitter 70a..d is optically coupled with a different one of the plurality of interference filters 56a..d. It is also envisaged that light emitters having substantially the same emission wavelength band profile may be all coupled to a same filter.

In this manner as the filters 56a..d are tilted the wavelength of light emitted from an associated emitter of a plurality of emitters 70a..d and passed by each filter of the plurality 56a..d for onward transmission to an analysing region 72 may be swept backwards and forwards through a desired range.

Also forming a part of the optical analyser 54 is a fibre optic bundle 74 for collecting light passed by the filters 56a..d. In the present embodiment the bundle 74 is configured with a plurality of branches 74a..d, each for collecting light passed by a different one of the filters 56a..d. Optionally, an optical coupling means, here illustrated as individual lenses 76a..d, may be provided to couple the light passed by each filter 56a..d into the fibre optic bundle 74.

Light so coupled exits the fibre optic bundle at an end 78 and enters the analysis region 72 which here is located between the end 78 and detection means 80 and in which a body part, such as a finger or an ear lobe (not shown), may be introduced. In an alternate embodiment the analysis region 72 may be configured to receive a blood sample confined in a cuvette, or other suitable holder or on a carrier such as a test strip transparent slide or other support surface.

In the present embodiment it is intended that light transmitted through the sample is to be detected by the detection means 80 and a signal representative of the intensity of the so detected light is to be passed to a data processor within a computing element 82. The data processor is configured to manipulate the signal in a known manner using an appropriate chemometric model to provide analysis results for a user as discussed above with reference to the computer 12 of Fig 1.

Also connected to the computing element 82 is a control unit 84 for the light source 70a..d and is configured to energise the emitters 70a..d in manner, such as sequentially, group-wise or individually in a non-sequential manner, dependent on control signals output from the computing element 82 and the type of analysis to be made.

The angular position of the shaft 58 may be monitored using elements well known in the art and provided to the computing element 82. Such elements may be, for example and without limitation, a shaft encoder associated with the shaft 58 or a position sensor associated with the drive plate 68 or a pulse counter associated with a stepper motor drive element (if employed) to count drive pulses sent to the motor. From this a determination of angle of tilt of the plurality of filters 56a..d may be made and hence the wavelength being passed by each illuminated filter 56a..d can be readily calculated in the computing element 82. As will be appreciated, the intensity of transmitted light detected by the detection means 80 can be then easily indexed with the incident wavelength and a transmission spectrum can be constructed.

It will be appreciated that similar position sensors can be provided and similar calculations then made to construct a reflection spectrum within the computer 12 of the optical analyser 2 of the first embodiment illustrated in Figs 1 to 3.

## Claims

1. An optical blood analyte monitor (2;54) comprising a near infra-red light source and complementary detection (6;80) means; a component analyser (12;82) having access to a chemometric model linking spectral features to a level of blood analyte of interest and configured to apply the model to signals received from the detection (6;80) means; **characterised in that** t the monitor further comprises a tilting filter arrangement (8;52) having a plurality of optical interference filters (20a..e;56a..d), each filter being tiltable to vary a wavelength of incident light from the source transmitted there through **and in that** the light source comprises a plurality of light emitters (4a..e;70a..d) selected to emit light in at least a wavelength region characteristically absorbed by blood components indicative of the analyte of interest and each being arranged to emit light along a different associated light-path (16a..e) in which is located an associated different one of the plurality of the interference filters (20a..e) and towards a same analysing region (18;72).

2. A monitor (2;54) as claimed in Claim 1 **characterised in that** the filters (20a..e;56a..d) of the plurality are reciprocatively tiltable.

3. A monitor (2;54) as claimed in Claim 1 or Claim 2 **characterised in that** the tilting filter arrangement (8;52) is provided with a carrier (44;66) and follower (46c;64) drive to effect a simultaneous tilting of all filters (20a..e;56a..d) of the plurality.

4. A monitor (2) as claimed in Claim 3 **characterised in that** the tilting filter arrangement (8) comprises a rotatable axle (42) having a rotational axis (14) **and in that** the arrangement further comprises a carrier (44) located on the axle (42) and a co-operable follower (46c) mechanically connected to an associated filter (20c) to effect the tilting thereof as the carrier (44) interacts with the follower (46c) on rotation of the axle (42).

5. A monitor (2) as claimed in Claim 4 **characterised in that** the filters (20a..e) are located angularly spaced apart about said axle (42) in a common plane.

6. A monitor (2) as claimed in Claim 5 **characterised in that** the rotatable axle (42) comprises a body portion (28) extending along the rotational axis (14) **and in that** the plurality of light emitters (4a..e) are relatively orientated to provide associated light-paths (16a..e) which intersect at the analysing region (18) beyond the body portion (28).

7. A monitor (2) as claimed in Claim 6 **characterised in that** the analyser further comprises a light pipe (26) for communicating illumination towards an optical sensor (6) after its interaction with the volume to be analysed.

8. A monitor (2) as claimed in Claim 7 **characterised in that** the body portion (28) is formed as hollow body having internal surfaces (30) delimiting the light pipe (26).

9. A monitor (2;54) as claimed in any preceding claim **characterised in that** t the plurality of light emitters (4a..e;70a..d) are selected to emit in wavelength regions preferentially absorbed by glucose containing material **and in that** component analyser (12;82) is provided with access to a chemometric model linking the spectral features to blood glucose levels.
